# EUROPÄISCHE PATENTANMELDUNG

(11) **EP 3 173 062 A1**
(43) Veröffentlichungstag der Anmeldung: **31.05.2017**
(21) Anmeldenummer: 15197159.5
(22) Anmeldetag: 30.11.2015
(51) Int. Cl.: A61K 6/00, A61C 13/15

(54) **POLYMERISATIONSLAMPE UND KOMPOSITE ZUR FÜLLUNG VON ZAHNKAVITÄTEN**

(71) Anmelder: PHILIPPS-UNIVERSITÄT MARBURG, 35037 Marburg (DE)
(72) Erfinder: Gente, Michael, 35091 Cölbe (DE); Kusch, Malte, 31134 Hildesheim (DE)
(74) Vertreter: Stumpf, Peter

(57) **Zusammenfassung**

Der Gegenstand der Erfindung sind neue lichthärtbare Komposite oder Kompositzusammensetzungen zur Füllung von Zahnkavitäten unter Verwendung einer neuen Polymerisationslampe (Aushärtungslampe). Die neuen Komposite oder Kompositzusammensetzungen sind zweizeitig polymerisierbar, da sie in geringer Menge einen ersten Initiator und in größerer Menge mindestens einen weiteren Initiator enthalten. Der erste Initiator reagiert auf andere Lichtwellenlängen als der mindestens eine weitere Initiator. Bei Bestrahlung mit Licht für den ersten Initiator wird das Komposit in den Gelzustand überführt (Vorhärtung), bei anschließender Bestrahlung mit dem Licht für den mindestens einen weiteren Initiator härtet das Komposit komplett aus (Endhärtung).

Der Gegenstand der Erfindung ist ebenso eine neue Polymerisationslampe, welche die für die mindestens zwei Initiatoren notwendigen Lichtwellenlängen zu den erforderlichen Zeiten in der erforderlichen Intensität abstrahlt. Bei der erfindungsgemäßen Anwendung der zweizeitig polymerisierbaren Komposite oder der zweizeitig polymerisierbaren Kompositzusammensetzungen und der neuen Polymerisationslampe werden polymerisationsbedingte Spannungen in Zahn und Füllung minimiert.

## Beschreibung

Die vorliegende Erfindung betrifft neue lichthärtbare Komposite oder Kompositzusammensetzungen zur Füllung von Zahnkavitäten unter Verwendung einer neuen Polymerisationslampe (Aushärtungslampe). Die neuen Komposite oder Kompositzusammensetzungen sind zweizeitig polymerisierbar, in der Form, dass sie mit mindestens zwei unterschiedlichen Lichtwellenlängen (Wellenlängen, Wellenlängenbereiche) der neuen Polymerisationslampe zu mindestens zwei unterschiedlichen Zeiten und über gleiche oder unterschiedliche Zeitlängen gehärtet werden. Die neuen Komposite oder Kompositzusammensetzungen werden mit der neuen Polymerisationslampe zunächst vom plastischen (fließfähigen, viskösen, flüssigen) Zustand durch die Beleuchtung mit Licht mit einer ersten Wellenlänge in einen Gelzustand überführt (Vorhärtung) und im Anschluss daran durch die Beleuchtung mit Licht mit einer zweiten Wellenlänge vollständig polymerisiert d.h. ausgehärtet (Endhärtung). Ermöglicht wird dies durch mindestens zwei unterschiedliche Initiatoren (Photoinitiatoren) in den neuen Kompositen oder Kompositzusammensetzungen. Diese Initiatoren sind durch verschiedene Lichtwellenlängen aktivierbar. Die neue Polymerisationslampe strahlt die dafür notwendigen Lichtwellenlängen zu verschiedenen Zeiten in der erforderlichen Intensität ab. Bei Anwendung der neuen Komposite oder Kompositzusammensetzungen und der neuen Polymerisationslampe wird die Zeit reduziert, die ein Zahnarzt braucht, um eine Füllung in einer Kavität eines Zahnes zu applizieren. Gleichzeitig wird die Qualität der Füllung verbessert.

Durch die Verwendung der neuen zweizeitig polymerisierbaren Komposite oder Kompositzusammensetzungen und der neuen Polymerisationslampe wird ein Verfahren zur schnelleren und spannungsärmeren Füllung von Zahnkavitäten mit Kompositen ermöglicht.

### Beschreibung des allgemeinen Gebietes der Erfindung und Stand der Technik

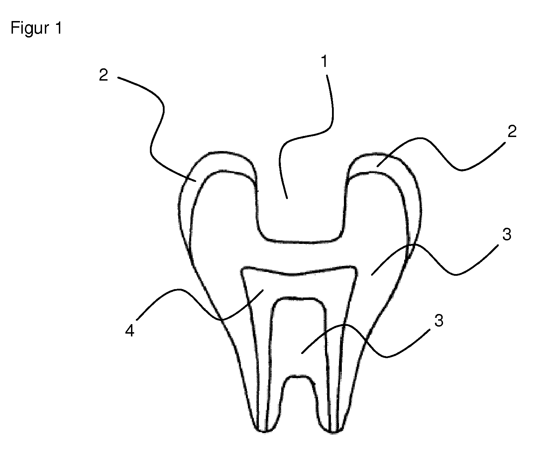

Die Karies ist eine Zahnerkrankung, bei der das Zahnhartgewebe, also der Zahnschmelz und das Dentin (=Zahnbein) geschädigt sind. Die Therapie besteht in einem ersten Schritt darin, alles erkrankte kariöse Gewebe zu entfernen (Exkavation). Der dabei entstehende Zahndefekt wird als Zahnkavität oder kurz Kavität bezeichnet. Die Figur 1 zeigt schematisch einen Längsschnitt durch einen Zahn mit einer Kavität, nachdem das kariöse Gewebe entfernt wurde.

Als zweiter Schritt der Therapie wird die Kavität mit einer Füllung gefüllt (Füllungstherapie, konservierende oder restaurierende Therapie) und damit das entfernte, erkrankte kariöse Gewebe ersetzt, um dem betroffenen Zahn wieder seine ursprüngliche Form und Funktion zu geben. Als Füllung werden verschiedene Materialien verwendet, beispielsweise Gold, Goldlegierungen, Keramiken, Amalgame (Silberamalgam) oder Komposite.

Die Verwendung von Komposit für die Füllung von Kavitäten gilt als Standardtherapie. Komposite sind zahnfarbene plastische Zahnfüllungsmaterialien, die aus einer organischen Kunststoffmatrix (Harz, Harzanteil des Komposits) und aus anorganischen sowie ggf. organischen Füllkörpern zusammengesetzt sind. Eine Verbundphase ermöglicht die chemische Verbindung der organischen Kunststoffmatrix mit den anorganischen Füllkörpern mit silikatischer Oberfläche, im Wesentlichen durch eine Silanisierung.

Die organische Kunststoffmatrix (=Harz, Harzanteil des Komposits) besteht aus einem Gemisch von organischen Monomeren, welche als Dimethacrylate nach der Formel M-R-M aufgebaut sind. In der Regel wird Methacrylat (Acryl), beispielsweise Hydroxyethylmethacrylat (HEMA), Triethylenglycoldimethacrylat (TEGDMA), Bisphenolglycidylmethacrylat (BisGMA) oder Urethandimethacrylat (UDMA) verwendet. Die organische Kunststoffmatrix liegt zusammen mit den untergemischten Füllkörpern in einem plastischen (fließfähigen, viskösen, flüssigen) Zustand vor, damit Komposite in die Kavität hineingedrückt oder wie eine zähflüssige Flüssigkeit eingefüllt werden können. Die organische Kunststoffmatrix muss nach der Applikation in die Zahnkavität gehärtet werden. Die Härtung erfolgt durch eine Polymerisation der organischen Monomere.

Als organische Kunststoffmatrix (=Harz, Harzanteil des Komposits) können auch Ormocer®e (=organically modified ceramics) verwendet werden, deren Moleküle sich aus einer -Si-O-Si-Kette ableiten, wobei die noch freien Si-Bindungen mit organischen Resten verknüpft sind. Die Reste enthalten polymerisierbare Kohlenstoffdoppelbindungen, die wie bei einem rein organischen Monomer polymerisieren und so die Erhärtung bewirken. Als organische Kunststoffmatrix für die erfindungsgemäßen Komposite oder Kompositzusammensetzungen können sowohl die organischen Monomere als auch die auf Ormocer®-Basis hergestellten Harze alleine als auch Mischungen aus Ormocer®e mit organischen Monomeren verwendet werden.

Die anorganischen Füllkörper können Glas, Glaskeramiken, Silikate oder Siliziumdioxid sein, welche eine mittlere Partikelgröße bis 10 µm aufweisen. Sie sind im Wesentlichen für die Härte des Komposits verantwortlich. Die anorganischen Füllkörper erhöhen die mechanische Festigkeit des Komposits, verbessern die Bruchfestigkeit und verringern die Polymerisationsschrumpfung.

Komposite können nach ihrer Viskosität eingeteilt werden in fließfähige und stopfbare Komposite.

Eine andere Einteilung der Komposite unterscheidet zwischen chemisch härtenden und lichthärtenden Kompositen sowie dualhärtenden Kompositen. Chemisch härtende Komposite enthalten mindestens zwei Komponenten, wobei mit der Vermischung der Komponenten der Polymerisationsprozess der organischen Kunststoffmatrix gestartet wird, der zur Aushärtung des Komposits führt. Lichthärtende Komposite enthalten einen Initiator (=Photoinitiator, Starter, Radikalstarter) und einen Koinitiator. Durch die Bestrahlung mit blauem oder ultraviolettem Licht wird der Polymerisationsprozess der organischen Kunststoffmatrix gestartet, der zur Aushärtung des Komposits führt. Dualhärtende Komposite enthalten sowohl chemisch härtende Komponenten als auch einen Initiator für die Lichthärtung.

Üblicherweise werden in der Zahnmedizin lichthärtende Komposite verwendet. Als Initiator werden z.B. Kampherchinon, Lucerin, (Di-)Benzoyl-Peroxid, Phenyl-Propandiol oder Acylgermanium-Verbindungen verwendet. Das blaue bzw. ultraviolette Licht wird im Komposit von dem Initiator (beispielsweise Kampherchinon) absorbiert, in Folge dessen die Initiatormoleküle in Radikale zerfallen. Diese Radikale lösen dann zusammen mit dem Koinitiator die Polymerisation der Monomere der organischen Kunststoffmatrix in dem Komposit aus:
Die Doppelbindungen in der organischen Kunststoffmatrix, z.B. im TEGMA oder BisGMA werden durch die Radikale aufgespalten und es bilden sich neue Bindungen zwischen den einzelnen Molekülen. Dadurch entstehen lange Molekülketten und das Komposit wird fest (=es ist polymerisiert, gehärtet, ausgehärtet). Bei der Verfestigung (Aushärtung) des Komposits (Polymerisation) verringert sich der Abstand der Harzmoleküle zueinander, mit der Folge, dass das erhärtete Komposit weniger Volumen ausfüllt als das noch plastische Komposit (plastische Phase). Dieses Phänomen wird Polymerisationsschrumpfung genannt.

In der Folge versucht sich das gehärtete Komposit von der Kavitätenwand zu lösen, die Verklebung des gehärteten Komposits mit der Kavitätenwand wird durch Zug- und Scherspannungen belastet. Ist die Spannung im ausgehärteten Komposit größer als die Festigkeit des Verbundes (der Verklebung) Komposit-Zahn, dann löst sich das Komposit an einzelnen Stellen von der Kavitätenwand. Der Patient hat dann Schmerzen an dem betroffenen Zahn, Bakterien können in die entstandenen Spalten zwischen dem ausgehärteten Komposit und der Kavitätenwand einwandern und der Zahn erkrankt erneut an Karies.

Als Koinitiator wird bei lichthärtenden Kompositen in der Regel ein tertiäres Amin verwendet, wie beispielsweise Dimethylaminoethanol oder Ethyl-4-(Dimethylamino)benzoat (4-(N,N-dimethylamino)benzoic acid ethyl ester oder DMABEE).

Lichthärtende Komposite können zusätzlich zur organischen Kunststoffmatrix, den anorganischen Füllkörpern (Füllstoffe) und dem Initiator noch weitere Komponenten enthalten, beispielsweise Akzeleratoren (z.B. Toluidin) und Inhibitoren (z.B. Hydrochinon, Hydrochinonmonomethylether oder butyliertes Hydroxytoluol) der Polymerisation, UV-Stabilisatoren (z.B. Benzophenon, 2-Hydroxy-4-Methoxybenzophenon), Verdünner zur besseren Verarbeitbarkeit, Nanopartikel zur Verbesserung der Biegefestigkeit sowie Farben und Pigmente.

Für die Härtung der lichthärtenden Komposite ist eine Bestrahlung mit Licht einer geeigneten Wellenlänge (beispielsweise blau oder ultraviolett) über eine ausreichende Dauer (ca. 10 bis 40 Sekunden) notwendig. Die Wellenlänge des eingesetzten Lichtes und die Bestrahlungsdauer sind abhängig von der Art des verwendeten Komposits, von dem Initiator, von den Akzeleratoren und von den Inhibitoren und der Intensität des von der Polymerisationslampe abgestrahlten Lichtes. Für unterschiedliche lichthärtende Komposite werden also unterschiedliche Polymerisationslampen verwendet, die Licht mit einer bestimmten, auf das jeweilige Komposit angepassten Wellenlänge ausstrahlen.

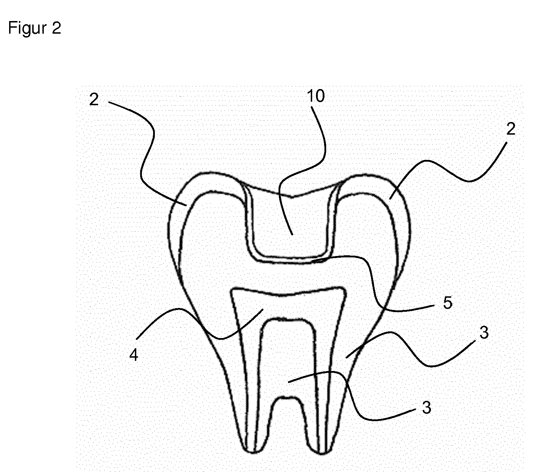

Die Füllungstherapie mit einem Komposit besteht im Wesentlichen aus folgenden Schritten:
1. Konditionierung (in Figur 2 nicht gezeigt): Die Konditionierung ist die Vorbereitung des Zahnschmelzrandes der Kavität und des Dentins durch eine Ätzung. Dadurch wird die spätere Verbindung zwischen Zahnschmelz und Dentin einerseits sowie dem Komposit andererseits verbessert. Für die Konditionierung wird beispielsweise 37-prozentige Phosphorsäure verwendet.
2. Priming, Grundierung (in Figur 2 nicht gezeigt): Ein Primer wird als Grundierung auf die Kavitätenoberfläche im Bereich des Dentins aufgebracht. Der Primer verbessert die spätere Verbindung zwischen Dentin und Komposit. Als Primer wird beispielsweise Hydroxyethylmethacrylat verwendet.
3. Bonding (in Figur 2 gezeigt): Ein Haftvermittler (Adhäsiv, Dentinadhäsiv) wird auf die Kavitätenoberfläche aufgebracht und bildet dadurch eine Bondingschicht (auch Hybridschicht genannt). Der Haftvermittler wird durch lichtinduzierte Polymerisation ausgehärtet. Der Haftvermittler dient als Kleber für das Komposit und stellt ein Verbindungselement zwischen dem Zahn (der Zahnkavität) und der Kompositfüllung dar. Als Haftvermittler wird beispielsweise ein dünnflüssiges Monomer verwendet. Der folgende Schritt 4 (Applikation des Komposits) wird erst ausgeführt, wenn die Bondingschicht ausgehärtet ist. Als Haftvermittler werden BisGMA, TEGDMA, UDMA, HEMA oder Mischungen dieser Monomere verwendet. Die Haftvermittler enthalten geeignete Initiatoren (Photoinitiatoren), damit sie durch lichtinduzierte Polymerisation aushärten können. Der Haftvermittler wird in einer sehr dünnen Schichtstärke auf die Kavitätenoberfläche aufgebracht, damit eine möglichst hohe Klebewirkung erreicht wird.
4. Applikation des Komposits: Das Komposit (Füllungskunststoff) wird im noch plastischen (viskösen, fließfähigen, flüssigen) Zustand in die Kavität hineingedrückt bzw. eingefüllt (in Figur 2 gezeigt). Die Menge des benötigten Komposits hängt von dem Volumen der Kavität ab. Das Komposit soll die Form und die Funktion des erkrankten Zahnes wieder herstellen. In Abhängigkeit von funktionellen und ästhetischen Gesichtspunkten werden unterschiedliche Komposite verwendet.
5. Härtung des Komposits: Bei chemisch härtenden Kompositen startet die Härtung (Polymerisation der organischen Kunststoffmatrix) dadurch, dass die mindestens zwei Komponenten des Komposits nach ihrer Vermischung als Folge einer chemischen Reaktion polymerisieren. Bei lichthärtenden Kompositen startet die Härtung (Polymerisation der organischen Kunststoffmatrix) dadurch, dass das von der Polymerisationslampe abgestrahlte Licht einer geeigneten Wellenlänge auf das Komposit fällt und dort vom Initiator absorbiert wird, wodurch der Initiator in ein Radikal zerfällt. Es findet also eine Aktivierung des Initiators statt. Das Radikal löst zusammen mit dem Koinitiator die Polymerisation der organischen Kunststoffmatrix aus, wodurch Polymere entstehen und das Komposit hart wird. Jeder Initiator reagiert bei einer bestimmten Wellenlänge und einer bestimmten Einstrahldauer des verwendeten Lichts. Üblicherweise ist die Härtung nach ca. 10 bis 40 Sekunden Bestrahlung mit dem geeigneten Licht abgeschlossen.
6. Polieren der freien Oberfläche des gehärteten Komposits.

Die Figur 2 zeigt einen schematischen Längsschnitt durch einen Zahn mit einer fertigen Kompositfüllung in einer Kavität gemäß des Standes der Technik.

Komposite weisen eine wesentliche nachteilige Eigenschaft auf, nämlich dass sie im Zuge der Härtung eine Volumenschrumpfung erleiden (Polymerisationsschrumpfung), welche ca. ein bis sechs Prozent des ursprünglichen Volumens beträgt. Diese Schrumpfung ist der wesentliche Nachteil einer Füllung mit einem Komposit: Da das Komposit auf Grund des Bondings (mit der dünnen Bondingschicht) mit der Kavitätenoberfläche fest verklebt ist, entstehen durch die Schrumpfung Zug- und Scherspannungen im Zahn. Diese Spannungen können große Schmerzen verursachen und sogar zur Bildung von Rissen und Spalten zwischen dem Zahn und der Kompositfüllung führen, wenn es zu einem Abriss der Füllung oder des Haftvermittlers (Bondingschicht, Adhäsivschicht) von der Oberfläche der Kavität gekommen ist. An diesen Stellen kann sich eine neue Karies entwickeln, so dass die gesamte Kompositfüllung wieder entfernt und eine neue Füllung angefertigt werden muss.

Bei Kompositen aus dem Stand der Technik härtet bei der Bestrahlung mit dem dafür vorgesehenem Licht erst die Oberfläche, die der Polymerisationslampe zugewandt ist. Die weiter von der Polymerisationslampe entfernt in der Tiefe der Kavität liegenden Anteile des Komposits härten erst mit einiger Zeitverzögerung. Da also das tiefer gelegene Komposit in einem weitgehend abgeschlossenem Raum härtet, können die durch die Schrumpfung induzierten Spannungen (Zug- und Scherspannungen) im Komposit nicht durch das Nachfließen von noch plastischem (viskösem, fließfähigem, flüssigem) Komposit ausgeglichen werden.

Die Polymerisationsschrumpfung des Komposits läuft in zwei Phasen ab: Während der Anfangsphase der Lichthärtung geliert das Komposit zu einer viskösen bis hochviskösen Masse, die jedoch noch nicht fest ist. Während dieser Phase findet der Großteil der Polymerisationsschrumpfung statt. In der anschließenden Härtungsphase härtet das Komposit aus. Während dieser zweiten Phase findet nur noch eine geringe Polymerisationsschrumpfung statt. Die Härtung geht von der Oberfläche des Komposits aus, die der Polymerisationslampe zugewandt ist und setzt sich in die Tiefe fort. Das Komposit erhärtet also in einem weitgehend abgeschlossenen Raum. Daher können die durch die Polymerisationsschrumpfung induzierten Spannungen nicht durch Nachfließen des Komposits ausgeglichen werden.

Es gibt mehrere Möglichkeiten im Stand der Technik, um diese Polymerisationsschrumpfung des Komposits auszugleichen:
1. Erhöhung des Anteils der anorganischen Füllkörper gegenüber dem Anteil der organischen Kunststoffmatrix (Harzanteil): Der Anteil der Füllkörper am Komposit kann zwischen 50 und 85 Volumenprozent betragen. Die Füllkörper sind gleichmäßig im Komposit verteilt und erhöhen die mechanische Festigkeit des Komposits. Da die Füllkörper nicht an der Polymerisationsreaktion während des Aushärtens teilnehmen und ihr Volumen während der Polymerisationsreaktion nicht abnimmt, wird durch einen hohen Anteil der Füllkörper im Komposit das Ausmaß der Polymerisationsschrumpfung verringert. Allerdings erhöhen die anorganischen Füllkörper den Elastizitätsmodul des ausgehärteten Komposits, so dass durch das Beimischen der anorganischen Füllkörper die Polymerisationsschrumpfung von Komposit zwar verringert werden kann, wegen des erhöhten Elastizitätsmoduls die Schrumpfungskräfte (Zug- und Scherspannungen) sich mit dieser Methode aber nicht wirkungsvoll reduzieren lassen.
2. Die Kompositfüllung wird schichtweise aus kleinen Portionen aufgebaut: Das Komposit wird in kleinen Portionen in die Kavität eingebracht, wobei jede einzelne Portion nach dem Einbringen lichtgehärtet wird. Dabei müssen die Portionen so eingebracht werden, dass ein günstiges Verhältnis von ungebundener Oberfläche des Komposits zu gebundener Oberfläche (=Oberfläche des Komposits, das am Zahn oder anderen Füllungsanteilen anhaftet) erzeugt wird, was zu einer Verringerung der Schrumpfungskräfte führt. Risse und Spalten, die bei der Polymerisationsschrumpfung der jeweils vorhergehenden Portion aufgetreten sind, werden durch die jeweils folgende Portion wieder gefüllt. Das ist die Standardverarbeitungstechnik in der Zahnmedizin, die jedoch viel Zeit benötigt, weil die Modellierung der Füllung immer wieder für das zwischenzeitliche Lichthärten unterbrochen werden muss.
   Dieses Vorgehen hat die Nachteile, dass es umständlich ist, dass der Patient eine längere Behandlungszeit ertragen muss und dass die Arbeitszeitkosten des Zahnarztes hoch sind.
3. Vorhärten des Komposits während der Applikation: Gleichzeitig mit dem Einbringen des noch plastischen (viskösen, fließfähigen, flüssigen) Komposits wird dieses durch eine der Fließgeschwindigkeit angepasste Lichtstärke bestrahlt, sodass eine Vorhärtung erfolgt. Der Polymerisationsprozess wird also schon während des Einbringens des Komposits eingeleitet, was zu einer erheblichen Reduktion der Schrumpfungskräfte führt. Während der Vorhärtung erfolgt der größte Teil der Polymerisationsschrumpfung. Während dieser Zeit kann das noch plastische (visköse, fließfähige, flüssige) Komposit nachlaufen, wodurch die Polymerisationsschrumpfung ausgeglichen wird. Das Vorhärten des Komposits während der Applikation hat den Nachteil, dass eine spezielle Lampe verwendet werden muss, die mit der Applikationsvorrichtung, mit der das Komposit in die Kavität eingebracht wird, eine Einheit bildet.
4. Veränderung der Monomere im Komposit: Durch die Auswahl speziell angepasster Monomere auf der Basis von Siloxan- und Oxiranverbindungen im Komposit kann das Schrumpfungsverhalten während des Aushärtens beeinflusst werden. Die Nachteile dieser Monomere sind eine verringerte Festigkeit des ausgehärteten Komposits und die Notwendigkeit, dass für diese Monomere ein spezieller Haftvermittler (Bonding) nötig ist, der nur von einem Hersteller verfügbar ist und für andere Komposite nicht eingesetzt werden kann.

### Aufgabe

Mit der vorliegenden Erfindung sollen eine Polymerisationslampe und darauf abgestimmte Komposite oder Kompositzusammensetzungen zur Verfügung gestellt werden, mit denen die bei der Polymerisation von Komposit in der Zahnkavität entstehende Schrumpfung verringert werden soll. Damit sollen die bei der Schrumpfung entstehenden Spannungen (Scher- und Zugspannungen) in der Kompositfüllung reduziert werden. Durch diese Reduktion der Spannungen in der Kompositfüllung sollen auch die Spannungen im Zahn verringert werden, welche große Schmerzen verursachen können. Ebenso soll die auf Grund der Polymerisationsschrumpfung entstehende Bildung von Rissen und Spalten zwischen dem Zahn und der Füllung verhindert werden.

### Lösung der Aufgabe

Die erfindungsgemäße Aufgabe wird gelöst durch die in den Ansprüchen genannte Polymerisationslampe und die in den Ansprüchen genannten Komposite oder Kompositzusammensetzungen. Die Polymerisationslampe erlaubt es, die in die Kavität eingebrachten Komposite oder Kompositzusammensetzungen mit unterschiedlichen Wellenlängen des Lichtes nacheinander zu bestrahlen. Dadurch härten die in die Kavität eingebrachten Komposite oder Kompositzusammensetzungen nacheinander oder zeitlich versetzt durch Polymerisation aus. Auf Grund dieser zeitlich versetzten Aushärtung (zweizeitige Aushärtung) ist immer plastisches (visköses, fließfähiges, flüssiges) Komposit zum Ausgleich des polymerisationsbedingten Volumenschwundes vorhanden und so entstehen keine schrumpfungsbedingten Spannungen in der Kompositfüllung und im Zahn. Um die erfinderische Idee zu verwirklichen, ist es essentiell, dass die eingesetzten Komposite oder Kompositzusammensetzungen und die Polymerisationslampe in spezifischer Weise aufeinander abgestimmt sind.

Der erfinderische Gedanke besteht aus folgenden Merkmalen:
- Die neuen Komposite oder Kompositzusammensetzungen enthalten mindestens zwei verschiedene Initiatoren, die durch Licht aktivierbar sind. Dadurch erhält man zweizeitig lichthärtbare Komposite oder Kompositzusammensetzungen, die dadurch gekennzeichnet sind, dass die mindestens zwei verschiedenen Initiatoren durch unterschiedliche Lichtwellenlängen aktiviert werden. Dadurch wird bewirkt, dass die erfindungsgemäßen Komposite oder Kompositzusammensetzungen erst dann vollständig aushärten, wenn die mindestens zwei verschiedenen Initiatoren durch unterschiedliche Lichtwellenlängen aktiviert worden sind. Es ist dabei wesentlich, dass ein erster Initiator in einer derart geringen Konzentration dem Komposit oder der Kompositzusammensetzung beigegeben ist, dass dieser erste Initiator bei der Bestrahlung mit der auf ihn abgestimmten Lichtwellenlänge das Komposit oder die Kompositzusammensetzung nicht vollständig härtet, sondern nur in einen zähen, gelartigen Zustand überführt, also eine Vorhärtung auslöst.
- Der mindestens eine weitere Initiator wird durch Licht mit einer anderen Wellenlänge als der erste Initiator aktiviert. Dadurch wird bewirkt, dass die erfindungsgemäßen Komposite oder Kompositzusammensetzungen nicht bereits bei der Bestrahlung mit Licht mit der ersten Wellenlänge vollständig aushärten, sondern erst, wenn das Licht mit der zweiten Wellenlänge, welche auf den mindestens einen weiteren Initiator abgestimmt ist, auf das Komposit oder die Kompositzusammensetzung gestrahlt wird. Dabei ist wesentlich, dass die Konzentration des mindestens einen weiteren Initiators so hoch ist, dass bei der Bestrahlung mit Licht der zweiten Wellenlänge über die Aktivierung des mindestens einen weiteren Initiators das Komposit oder die Kompositzusammensetzung vollständig aushärtet (Endhärtung).
- Die erfindungsgemäßen Komposite oder Kompositzusammensetzungen bestehen aus einem einzigen Komposit mit mindestens zwei verschiedenen Initiatoren oder aus einem Gemisch von zwei oder mehreren verschiedenen Kompositen (Kompositzusammensetzung) mit mindestens zwei verschiedenen Initiatoren. Es ist wesentlich, dass die erfindungsgemäßen Komposite oder Kompositzusammensetzungen mindestens zwei verschiedene Initiatoren enthalten, die durch Licht mit unterschiedlichen Wellenlängen aktivierbar sind, so dass die Komposite oder Kompositzusammensetzungen zweizeitig lichthärtbar sind.
- Wenn nur ein erfindungsgemäßes Komposit verwendet wird, dann muss dieses mindestens zwei verschiedene Initiatoren enthalten. In diesem Fall bewirkt der erste Initiator durch die Bestrahlung mit Licht einer ersten Wellenlänge nur eine unvollständige Härtung des Komposits (Vorhärtung). Anschließend bewirkt der mindestens eine weitere Initiator durch die Bestrahlung mit Licht einer zweiten Wellenlänge die eigentliche Härtung (Endhärtung) des erfindungsgemäßen Komposits.
- Die erfindungsgemäße Polymerisationslampe ist geeignet, Licht mit mindestens zwei verschiedenen Wellenlängen, jeweils angepasst an die mindestens zwei verschiedenen Initiatoren, nacheinander, zeitversetzt oder gleichzeitig abzustrahlen. Dadurch wird bewirkt, dass durch das Licht mit der ersten Wellenlänge nur der erste Initiator aktiviert wird und durch das Licht mit der zweiten Wellenlänge nur der mindestens eine weitere Initiator aktiviert wird. Dabei ist es wesentlich, dass das Licht mit der ersten Wellenlänge und das Licht mit der zweiten Wellenlänge so einander überlagert werden, dass sie mit einem Lichtleiter 25 zum Komposit oder zur Kompositzusammensetzung geleitet werden können. Diese Überlagerung wird durch eine geeignete optische Vorrichtung 24 erreicht, beispielsweise durch einen dichroitischen Spiegel oder ein dichroitisches Strahlteilerprisma. Es ist dabei wesentlich, dass die geeignete optische Vorrichtung 24 so angeordnet ist, dass sie verhindert, dass die unerwünschten Wellenlängenanteile des Lichtes mit der ersten Wellenlänge auf das Komposit oder die Kompositzusammensetzung treffen. Das heißt, dass diese unerwünschten Wellenlängenanteile nicht über den Lichteintrittsbereich 26, den Lichtleiter 25 und den Lichtaustrittsbereich 27 auf das Komposit oder die Kompositzusammensetzung geleitet werden. Die unerwünschten Wellenlängenanteile des ersten Lichtes mit der ersten Wellenlänge sind jene Wellenlängenanteile, welche das Potential haben, den mindestens einen weiteren Initiator zu aktivieren. Es muss auf jeden Fall verhindert werden, dass der mindestens eine weitere Initiator bereits durch Wellenlängenanteile des Lichtes mit der ersten Wellenlänge aktiviert wird, weil dadurch die vollständige Härtung des Komposits oder der Kompositzusammensetzung zu früh stattfinden würde.
- Werden mehr als zwei Initiatoren verwendet, dann muss die Polymerisationslampe auch Licht mit weiteren Wellenlängen ausstrahlen können.

Insbesondere beschreibt die vorliegende Erfindung Komposite oder Kompositzusammensetzungen, darauf abgestimmte Initiatoren, Akzeleratoren und Inhibitoren, geeignet für die Polymerisation der Komposite oder Kompositzusammensetzungen, sowie eine Polymerisationslampe mit einer besonderen Optik. Die besondere Optik der Polymerisationslampe strahlt Licht mit unterschiedlichen Wellenlängen aus, wobei die Abstrahlung des Lichtes für die verschiedenen Wellenlängen in gleicher Weise erfolgt. Dabei kann jede Wellenlänge zu unterschiedlichen Zeiten und in unterschiedlicher Dauer und mit unterschiedlicher Flächenenergiedichte auf das Komposit oder die Kompositzusammensetzung gestrahlt werden. In Kombination mit der erfindungsgemäßen Polymerisationslampe und in Abhängigkeit von den erfindungsgemäßen Initiatoren, Akzeleratoren und Inhibitoren werden die erfindungsgemäßen Komposite oder Kompositzusammensetzungen in einer zeitlichen Reihenfolge nacheinander erst vorgehärtet (Vorhärtung) und anschließend vollständig ausgehärtet (Endhärtung). Die erfindungsgemäßen Komposite oder Kompositzusammensetzungen können auch eine Mischung aus zwei oder mehr Kompositen sein, welche sich in der Art und Menge der beigemischten Initiatoren, Akzeleratoren und Inhibitoren voneinander unterscheiden.

Die erfindungsgemäße Aushärtungslampe und die erfindungsgemäßen Komposite oder Kompositzusammensetzungen können mit einer Vorrichtung kombiniert werden, die während der Applikation des Komposits oder der Kompositzusammensetzungen in die Kavität gleichzeitig durch Licht mit einer geeigneten Wellenlänge das Komposit oder die Kompositzusammensetzung vorhärtet. Das ergibt noch spannungsärmere und damit hochwertigere Kompositfüllungen.

Die erfindungsgemäßen zweizeitig lichthärtbaren Komposite oder Kompositzusammensetzungen sind geeignet zur Füllung einer Zahnkavität, wodurch eine Kompositfüllung entsteht. Sie umfassen eine organische Kunststoffmatrix, anorganische oder organische Füllkörper, mindestens einen Koinitiator, Akzeleratoren, Inhibitoren, UV-Stabilisatoren, Verdünner, Nanopartikel, Farben, Pigmente und einen ersten Initiator, der durch Licht eines ersten Wellenlängenbereiches aktivierbar ist. Sie sind dadurch gekennzeichnet, dass das Komposit oder die Kompositzusammensetzung mindestens einen weiteren Initiator umfasst, der durch Licht eines weiteren Wellenlängenbereiches aktivierbar ist, wobei der weitere Wellenlängenbereich unterschiedlich zum ersten Wellenlängenbereich ist.

Die erfindungsgemäßen zweizeitig lichthärtbaren Komposite oder Kompositzusammensetzungen sind dadurch gekennzeichnet, dass der erste und der mindestens eine weitere Initiator ausgewählt ist aus der Gruppe Kampherchinon, Lucerin, (Di-)Benzoyl-Peroxid, Phenyl-Propandiol oder Acylgermanium-Verbindungen.

Die erfindungsgemäßen Komposite oder Kompositzusammensetzungen sind zweizeitig lichtpolymerisierbare (lichthärtbare) Komposite, welche aus mindestens einer organischen Kunststoffmatrix (polymerisierbare Harzmatrix) und aus anorganischen Füllkörpern zusammengesetzt sind. Die erfindungsgemäßen Komposite oder Kompositzusammensetzungen sind durch mindestens zwei verschiedene Initiatoren (Lichtinitiatoren) gekennzeichnet, die auf verschiedene Wellenlängen ansprechen. Als Beispiel können die erfindungsgemäßen Komposite oder Kompositzusammensetzungen als Initiatoren Lucerin und Kampherchinon enthalten. Die erfindungsgemäße Neuentwicklung besteht darin, dass von dem ersten Lichtinitiator (z.B. vom Kampherchinon) nur eine solche geringe Menge dem Komposit oder der Kompositzusammensetzung beigegeben wird, die nicht für eine vollständige Aushärtung des Komposits oder der Kompositzusammensetzung ausreicht. Von dem mindestens einem weiteren Lichtinitiator (z.B. vom Lucerin) hingegen wird eine solche große Menge beigegeben, die zur vollständigen Aushärtung des Komposits oder der Kompositzusammensetzung ausreicht. Die erfindungsgemäßen Komposite oder Kompositzusammensetzungen werden gemäß des Standes der Technik in die Kavität eingebracht.

Nach dem Einbringen in die Kavität wird durch die Bestrahlung mit Licht mit einer ersten Wellenlänge der erste Initiator aktiviert (z.B. bei der Verwendung von Kampherchinon mit Licht einer Wellenlänge von 460 nm über eine Dauer von 15 Sekunden und einer Energiedichte von 800 mW/cm²). Da die Menge des ersten Initiators so gewählt wird, dass sie gerade ausreicht, um die Anfangsphase der Lichthärtung einzuleiten, geliert das Komposit oder die Kompositzusammensetzung zu einer viskösen bis hochviskösen Masse, wird aber noch nicht fest (Vorhärtung). Auch bei einer hohen Lichtdosis wird das Komposit oder die Kompositzusammensetzung nicht fest. In diesem Zustand ist das Komposit oder die Kompositzusammensetzung schon zu einem großen Teil geschrumpft, kann aber mangels Festigkeit noch keine Schrumpfungskräfte aufbauen. Die entstandenen Schrumpfungen können durch Kriechen des noch viskösen bis hochviskösen Komposits oder der noch viskösen bis hochviskösen Kompositzusammensetzung noch ausgeglichen werden, ohne dass sich nennenswerte Kräfte aufbauen.

Der Initiator Lucerin kann durch Licht mit einer Wellenlänge von 460 nm nicht aktiviert werden, da die Quantenenergie zu gering ist.

Anschließend wird das Komposit oder die Kompositzusammensetzung mit Licht einer zweiten Wellenlänge bestrahlt (z.B. bei der Verwendung von Lucerin als mindestens einen weiteren Initiator mit Licht einer Wellenlänge von 400 nm über eine Dauer von 15 Sekunden und einer Energiedichte von 800 mW/cm²). Dadurch erreicht das Komposit oder die Kompositzusammensetzung seine Endhärte, denn von dem mindestens einen weiteren Initiator, beispielsweise dem Lucerin, ist die für die Endhärtung notwendige Menge im Komposit oder in der Kompositzusammensetzung enthalten.

Die Verwendung von mindestens zwei verschiedenen Initiatoren in einem Komposit oder in einer Kompositzusammensetzung hat den Effekt, dass die Aushärtung des Komposits oder der Kompositzusammensetzung in zwei zeitlich aufeinanderfolgenden Schritten stattfindet (zweizeitig polymerisierbares Komposit oder Kompositzusammensetzung). Nach der gesamten Füllung der Zahnkavität durch das erfindungsgemäße Komposit oder durch die erfindungsgemäße Kompositzusammensetzung wird durch die Aktivierung des nur in geringer Menge vorhandenen ersten Initiators eine Vorhärtung ausgelöst. Im Zuge dieser Vorhärtung findet der größte Teil der Gesamtschrumpfung statt. Wegen der noch fehlenden Festigkeit des Komposits oder der Kompositzusammensetzung werden dabei jedoch kaum Schrumpfungsspannungen aufgebaut. Während der Endhärtung findet nur ein geringer Teil der Gesamtschrumpfung statt. Daher sind nach dem Abschluss der Endhärtung kaum schrumpfungsbedingte Spannungen vorhanden.

Ein zweizeitig polymerisierbares Komposit oder eine zweizeitig polymerisierbare Kompositzusammensetzung mit mindestens zwei verschiedenen Initiatoren eignet sich besonders für sogenannte Bulkfill-Komposite, die in Schichtstärken von bis zu vier und mehr Millimeter verarbeitet werden können.

Prinzipiell ist man frei in der Wahl, welcher Initiator in der niedrigeren und welcher Initiator in der höheren Konzentration dem erfindungsgemäßen Komposit oder der erfindungsgemäßen Kompositzusammensetzung beigegeben wird. Von Vorteil ist es, den Initiator in der geringeren Konzentration zu verwenden, der die geringere Quantenenergie zur Aktivierung benötigt. Damit kann sicher ausgeschlossen werden, dass die zur Lichthärtung verwendete Lichtwellenlänge für den ersten Initiator unbeabsichtigt auch den mindestens einen weiteren Initiator aktiviert.

Um das erfindungsgemäße, zweitzeitig polymerisierbare Komposit oder die zweizeitig polymerisierbare Kompositzusammensetzung auszuhärten (Vorhärtung und Endhärtung), ist eine Polymerisationslampe erforderlich, welche die passenden Lichtwellenlängen über die notwendigen Zeiträume abstrahlen kann, so dass durch die erste Lichtwellenlänge nur der erste Initiator aktiviert wird und erst dann durch die mindestens eine weitere Lichtwellenlänge der mindestens eine weitere Initiator aktiviert wird. Diese spezielle Polymerisationslampe ist Teil der Erfindung.

Die erfindungsgemäßen Komposite oder der erfindungsgemäßen Kompositzusammensetzungen können auch in zwei Schichten für die Füllung einer Kavität eingesetzt werden, wobei eine Schicht eine Ausgleichsschicht darstellt und die zweite Schicht die eigentliche Füllung der Kavität 1 darstellt.

Ebenfalls können die erfindungsgemäßen Komposite oder der erfindungsgemäßen Kompositzusammensetzungen aus einer Mischung von mindestens zwei verschiedenen Kompositzusammensetzungen mit unterschiedlicher Viskosität bestehen. Wesentlich ist, dass die verschiedenen Kompositzusammensetzungen ebenfalls mindestens zwei verschiedene Initiatoren enthalten, damit die Kompositzusammensetzungen zweizeitig polymerisierbar sind.

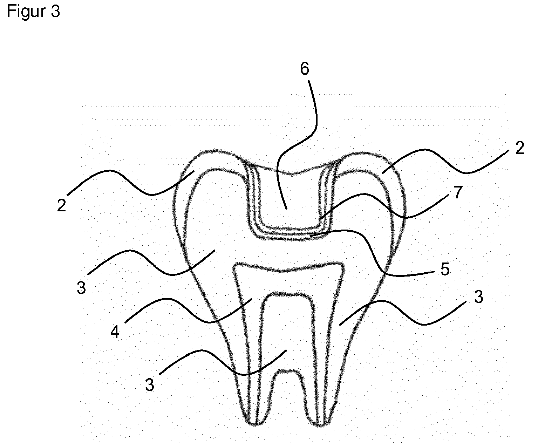

In einem Ausführungsbeispiel der Erfindung wird das zweizeitig polymerisierbare Komposit oder die Kompositzusammensetzung als Ausgleichsschicht verwendet, wie in Figur 3 dargestellt: Die Ausgleichsschicht 7 ist ein Komposit oder eine Kompositzusammensetzung mit einer geringen Viskosität und mit einem ersten Initiator, beispielsweise Lucerin. Dieses Komposit oder diese Kompositzusammensetzung der Ausgleichsschicht 7 wird als dünne Schicht lückenlos auf die Kavitätenwand aufgebracht. Anschließend wird das Komposit oder die Kompositzusammensetzung 6 der eigentlichen Füllung der Kavität mit mindestens einem weiteren Initiator, beispielsweise Kampherchinon in die Kavität eingebracht und damit die Kavität gefüllt. Für die Härtung des Komposits oder der Kompositzusammensetzung 6, welches die eigentliche Füllung darstellt, wird mit der erfindungsgemäßen Polymerisationslampe Licht mit einer Wellenlänge von 460 nm auf das Komposit oder die Kompositzusammensetzung gestrahlt, wodurch der Initiator Kampherchinon nur die Polymerisation des Komposits oder der Kompositzusammensetzung 6 auslöst. Da bei dieser Wellenlänge des eingestrahlten Lichtes der Initiator Lucerin im Komposit oder in der Kompositzusammensetzung 7 der Ausgleichsschicht nicht aktiviert wird, bleibt bei der Härtung des Komposits oder der Kompositzusammensetzung 6 der eigentlichen Füllung das Komposit oder die Kompositzusammensetzung 7 der Ausgleichsschicht noch fließfähig. Es können sich also zwischen dem Komposit oder der Kompositzusammensetzung 6 der Füllung und der Kavitätenwand keine Spannungen aufbauen. Das ausgehärtete Komposit oder die ausgehärtete Kompositzusammensetzung 6 schwimmt in der dünnen Schicht des noch ungehärteten Komposits oder der ungehärteten Kompositzusammensetzung 7 der Ausgleichsschicht, so dass die Volumenschrumpfung des Komposits oder der Kompositzusammensetzung 6 beim Polymerisieren durch Nachfließen des noch ungehärteten Komposits oder der ungehärteten Kompositzusammensetzung 7 der Ausgleichsschicht kompensiert wird.

Erst wenn das Komposit oder die Kompositzusammensetzung 6 erhärtet ist, wird mit der erfindungsgemäßen Polymerisationslampe Licht mit einer zweiten Wellenlänge abgestrahlt, beispielsweise 400 nm, wodurch der mindestens eine weitere Initiator im Komposit oder in der Kompositzusammensetzung 7 der Ausgleichsschicht aktiviert wird und die Polymerisation des Komposits oder der Kompositzusammensetzung 7 der Ausgleichsschicht beginnt. Nach einer ausreichend langen Bestrahlungsdauer ist auch das Komposit oder die Kompositzusammensetzung 7 der Ausgleichsschicht vollständig gehärtet und das bereits gehärtete Komposit oder die Kompositzusammensetzung 6 wird in die Zahnkavität durch das Komposit oder die Kompositzusammensetzung 7 der Ausgleichsschicht einpolymerisiert. Da die Schichtstärke der Ausgleichsschicht 7 gering ist, entstehen bei der Polymerisation des Komposits oder der Kompositzusammensetzung 7 der Ausgleichsschicht kaum Spannungen.

Für diese Anwendung des erfindungsgemäßen zweizeitig polymerisierbaren Komposits oder der Kompositzusammensetzung ist eine dafür geeignete Polymerisationslampe nötig. Es ist wesentlich, dass die Polymerisationslampe unterschiedliche Lichtwellenlängen passend zu den unterschiedlichen Initiatoren in den unterschiedlichen Kompositen oder Kompositzusammensetzungen abstrahlt, damit die mindestens zwei verschiedenen Initiatoren nicht zur selben Zeit aktiviert werden, sondern nacheinander.

Damit beim Einbringen des Komposits oder der Kompositzusammensetzung 6 der Füllung das Komposit oder die Kompositzusammensetzung 7 der Ausgleichsschicht nicht von der Kavitätenwand verdrängt wird, kann nach dem Einbringen des Komposits oder der Kompositzusammensetzung 7 der Ausgleichsschicht dessen Viskosität erhöht werden. Dem Komposit oder der Kompositzusammensetzung 7 der Ausgleichsschicht wird eine geringe, genau abgestimmte Menge des mindestens einen weiteren Initiators, der auch im Komposit oder der Kompositzusammensetzung 6 der Füllung enthalten ist, beigemengt. Das Komposit oder die Kompositzusammensetzung 7 der Ausgleichsschicht wird durch kurzzeitige Bestrahlung mit Licht der für den mindestens einen weiteren Initiator im Komposit oder der Kompositzusammensetzung 6 der Füllung geeigneten Wellenlänge in einen zähen, gelartigen Zustand überführt (Vorhärtung). In diesem zähen, gelartigen Zustand ist das Komposit oder die Kompositzusammensetzung 7 der Ausgleichsschicht nicht mehr fließfähig, aber durch Kriechen noch in der Lage, die Schrumpfung des Komposits oder der Kompositzusammensetzung 6 der Füllung auszugleichen. Auch hier wird das Grundprinzip der zweizeitigen Polymerisation verwirklicht.

Diese Vorgehensweise hat den Vorteil, dass unter Umständen auf das Auftragen und damit auch auf die Härtung einer Bondingschicht 5 verzichtet werden kann. Das Komposit oder die Kompositzusammensetzung 7 der Ausgleichsschicht wird in diesem Fall nach der Konditionierung und dem Priming direkt auf die Kavitätenwand dünn und lückenlos aufgetragen und übernimmt, nachdem es in den zähen, gelartigen Zustand überführt worden ist, die Funktion des Bondings und gleichzeitig die Funktion der Ausgleichsschicht.

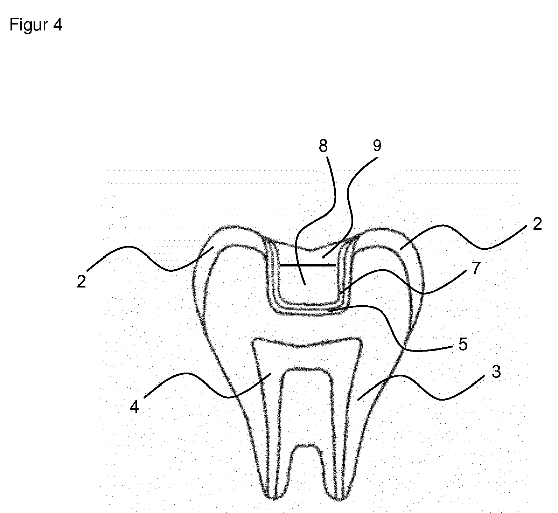

In einem weiteren Ausführungsbeispiel der Erfindung ist das zweizeitig polymerisierbare Komposit eine Kompositzusammensetzung eines fließfähigen selbstnivellierenden Komposits 8 und eines stopfbaren, zähen Komposits 9, wie in Figur 4 dargestellt. Nach dem Einbringen des Komposits oder der Kompositzusammensetzung der Ausgleichsschicht 7 mit einem ersten Initiator wird dieses durch die Bestrahlung mit Licht einer ersten Wellenlänge in den zähen, gelartigen Zustand überführt (Vorhärtung). Dann wird der Hauptteil der Kavität mit einem fließfähigen selbstnivellierenden Komposit 8 enthaltend mindestens einen weiteren Initiator aufgefüllt, und zwar derart, dass zur Mundhöhle hin noch 1 bis 2 mm der Kavität frei bleiben. Dann wird das Komposit 8 mit Licht einer zweiten Wellenlänge gehärtet, welches den mindestens einen weiteren Initiator im Komposit 8 aktiviert. Anschließend wird die Kavität mit einem stopfbaren, zähen Komposit 9 aufgefüllt, das sich für die Modellation der Kaufläche eignet. Das Komposit 9 enthält einen beliebigen Initiator, nur nicht den ersten Initiator des Komposits oder der Kompositzusammensetzung 7 der Ausgleichsschicht. Nach Abschluss der Modellation wird das Komposit 9 mit Licht einer geeigneten Wellenlänge gehärtet. Nach der vollständigen Aushärtung des Komposits 8 und des Komposits 9 wird das Komposit oder die Kompositzusammensetzung 7 der Ausgleichsschicht fertig gehärtet (Endhärtung), in dem es mit Licht der ersten Wellenlänge bestrahlt wird. Voraussetzung für die Härtung des Komposits oder der Kompositzusammensetzung 7 der Ausgleichsschicht ist, dass das Licht mit der ersten Wellenlänge in einer ausreichenden Lichtdosis eingestrahlt wird und dass die Komposite oder Kompositzusammensetzungen 8 und 9 für diese Wellenlänge ausreichend lichtdurchlässig sind. Alternativ kann das Komposit oder die Kompositzusammensetzung 7 der Ausgleichsschicht vor dem Einbringen des Komposits 9 gehärtet werden. In diesem Fall verursacht die Polymerisation des Komposits 9 Schrumpfungsspannungen, die aber wegen der geringen Schichtdicke nur gering ausfallen.

Diese Füllung der Kavität mit Komposit oder mit einer Kompositzusammensetzung 7 der Ausgleichsschicht und einer Kompositzusammensetzung bestehend aus einem selbstnivellierenden Komposit 8 und einem stopfbaren Komposit 9 ist besonders dafür geeignet, die Kaufläche des behandelten Zahnes in gewünschter Weise zu modellieren.

Für die zweizeitige Härtung der erfindungsgemäßen Komposite oder Kompositzusammensetzungen ist die erfindungsgemäß gestaltete Polymerisationslampe erforderlich. Nachdem das Aushärten der erfindungsgemäßen Komposite oder Kompositzusammensetzungen durch das Bestrahlen mit Licht der ersten Wellenlänge gestartet worden ist, wird anschließend Licht mit einer zweiten Wellenlänge abgestrahlt, wodurch der mindestens eine weitere Initiator in den erfindungsgemäßen Kompositen oder Kompositzusammensetzungen aktiviert wird. Der Zeitversatz und die Intensität der Bestrahlung mit Licht der mindestens zwei verschiedenen Wellenlängen müssen so aufeinander abgestimmt sein, dass die in der Tiefe der Kavität fortschreitende Härtung in Folge der Aktivierung des mindestens einen weiteren Initiators immer vor der Erhärtungsfront des Komposits oder der Kompositzusammensetzung 7 der Ausgleichsschicht liegt. Dann ist an den Stellen, an denen das Komposit oder die Kompositzusammensetzung 6, 8 oder 9 schrumpft, das Komposit oder die Kompositzusammensetzung 7 der Ausgleichsschicht immer noch im kriech- bzw. fließfähigen Zustand und kann somit die Schrumpfung des Komposits oder der Kompositzusammensetzung 6, 8 oder 9 ausgleichen. Es ist wesentlich, dass die Polymerisationslampe unterschiedliche Lichtwellenlängen passend zu den unterschiedlichen Initiatoren in den erfindungsgemäßen Kompositen oder Kompositzusammensetzungen abstrahlt, damit die mindestens zwei Initiatoren nicht zur selben Zeit aktiviert werden, sondern nacheinander.

Für die erfindungsgemäße Härtung des Komposits oder der Kompositzusammensetzung 7 der Ausgleichsschicht ist es essentiell, dass die zweizeitige Bestrahlung exakt an die Eigenschaften der zwei verwendeten Initiatoren angepasst ist. Daher muss für die erfindungsgemäße Härtung der Komposite oder der Kompositzusammensetzungen 6, 8 oder 9 und des Komposits oder der Kompositzusammensetzung 7 der Ausgleichsschicht eine Polymerisationslampe 100 verwendet werden, die eine exakte zweizeitige Bestrahlung ermöglicht und bei der am Lichtaustrittsbereich 27 das Licht beider Wellenlängen mit möglichst gleichmäßiger Intensitätsverteilung austritt. Die Intensitätsverteilungen im Nahfeld des Lichtaustrittsbereiches 27 der Polymerisationslampe 100 sollen für die mindestens zwei Lichtwellenlängenbereiche nahezu identisch sein. Dieses Ziel ist nur bedingt erreichbar, wenn mindestens zwei Lichtquellen nebeneinander in einer Baueinheit zu einer Polymerisationslampe angeordnet sind und in einen Lichtleiter einstrahlen, wie dies im Stand der Technik beschrieben wird. Bei Polymerisationslampen aus dem Stand der Technik können die verschiedenen Wellenlängen zwar nacheinander eingeschaltet werden, jedoch haben diese für die unterschiedlichen Wellenlängen eine ungleichmäßige Intensitätsverteilung im Nahfeld vor dem Lichtaustrittsbereich, da die einzelnen Lichtquellen nebeneinander liegen. Damit ist eine derart konstruierte Polymerisationslampe aus dem Stand der Technik für die erfindungsgemäße Anwendung nicht brauchbar.

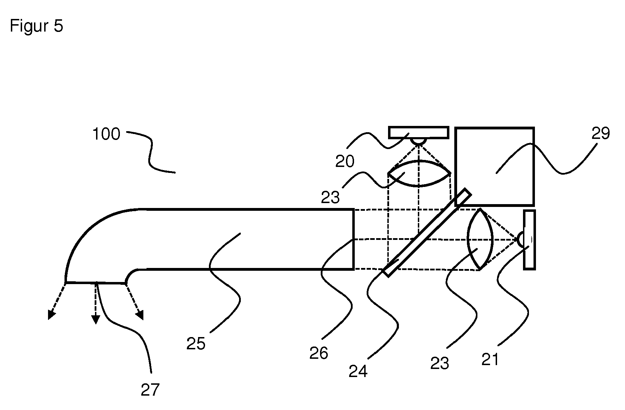

Es ist vorteilhaft, das Licht mit den mindestens zwei verschiedenen Wellenlängen in räumlich getrennten Lichtquellen 20, 21, 28 in einiger Entfernung zueinander zu erzeugen und über eine geeignete Optik zu bündeln, beispielsweise über eine Kondensor-Optik 23. Ebenso ist es vorteilhaft, das Licht mit den mindestens zwei verschiedenen Wellenlängen mittels eines geeigneten optischen Elementes 24 einander zu überlagern, beispielsweise mittels eines frequenzselektiven Strahlteilerprismas oder eines dichroitischen Strahlteilerprismas oder eines frequenzselektiven Spiegels oder eines dichroitischen Spiegels oder eines nichtfrequenzselektiven teildurchlässigen Spiegels. Das Licht mit den mindestens zwei verschiedenen Wellenlängen kann so in den Lichtleiter 25 oder direkt auf die Oberfläche der erfindungsgemäßen Komposite oder der erfindungsgemäßen Kompositzusammensetzungen eingestrahlt werden. Eine schematische Darstellung der erfindungsgemäßen Polymerisationslampe 100 mit der Führung des Lichts mit den mindestens zwei verschiedenen Wellenlängen (Strahlzusammenführung) findet sich in Figur 5.

Die erfindungsgemäße Polymerisationslampe 100 zur Aktivierung der Initiatoren in einem erfindungsgemäßen Komposit oder in einer erfindungsgemäßen Kompositzusammensetzung umfasst mindestens eine Lichtquelle 20, 21, 28 und ein optisches Element 24, das zwischen der mindestens einen Lichtquelle 20, 21, 28 und dem Lichtleiter 25 angeordnet ist. Das optisches Element 24 filtert das Licht der mindestens einen Lichtquelle 20, 21, 28 so, dass nur Anteile des Lichtes mit bestimmten, den verwendeten Initiatoren angepassten Wellenlängenbereichen passieren können. Ebenso umfasst die erfindungsgemäße Polymerisationslampe 100 eine Steuereinheit 29 zur zeitlichen Steuerung der mindestens einen Lichtquelle 20, 21, 28 und / oder des optischen Elementes 24 in Hinblick auf die abgestrahlte Lichtwellenlänge, den Zeitpunkt der Bestrahlung und die Dauer der Bestrahlung, so dass die Bestrahlung der Initiatoren in den erfindungsgemäßen Kompositen oder in den erfindungsgemäßen Kompositzusammensetzungen zeitlich gesteuert werden kann. Die Steuereinheit 29 steuert auch die Lichtstärke. Ebenso umfasst die erfindungsgemäße Polymerisationslampe 100 einen Lichtleiter 25 mit einem Lichteintrittsbereich 26 und einem Lichtaustrittsbereich 27, wobei der Lichtleiter 25 das Licht von der mindestens einen Lichtquelle 20, 21, 28 zum Komposit oder zu der Kompositzusammensetzung leitet.

Die erfindungsgemäße Polymerisationslampe 100 ist dadurch gekennzeichnet, dass der erste und der mindestens eine weitere Initiator im Komposit oder in der Kompositzusammensetzung einzeln nacheinander oder gemeinsam aktivierbar sind.

Als Lichtquellen 20, 21, 28 können Leuchtdioden verwendet werden. Es können auch Halogenbirnen oder Plasmalampen als Lichtquellen dienen, sofern deren Licht durch geeignete Filter (nicht in Figur 5 gezeigt) geleitet wird. Von den mindestens zwei Lichtquellen 20, 21, 28 wird das Licht mit den mindestens zwei verschiedenen Wellenlängen über dafür geeignete Optiken 23 und 24 geleitet, so dass es zum Lichteintrittsbereich 26 des Lichtleiters 25 gelangt. Über den Lichtleiter 25 wird das Licht mit den mindestens zwei verschiedenen Wellenlängen zum Lichtaustrittsbereich 27 geleitet, von wo es auf das zu härtende erfindungsgemäße Komposit oder auf die zu härtende erfindungsgemäße Kompositzusammensetzung fällt. In dem zu härtenden erfindungsgemäßen Komposit oder der zu härtenden erfindungsgemäßen Kompositzusammensetzung aktiviert das Licht mit den mindestens zwei verschiedenen Wellenlängen die mindestens zwei verschiedenen Initiatoren, wodurch die Polymerisation der erfindungsgemäßen Komposite oder Kompositzusammensetzungen ausgelöst wird und die erfindungsgemäßen Komposite oder Kompositzusammensetzungen aushärten.

Die Ausführung der Polymerisationslampe 100 gemäß Figur 5 hat den zusätzlichen Vorteil, dass durch die Zusammenführung des Lichtes verschiedener Wellenlängen über das optische Element 24 die Wellenlängen des Lichtes der Lichtquellen 20, 21, 28 zusätzlich eingegrenzt werden können. Diese Eingrenzung des Wellenlängenbereiches ist vorteilhaft, um zu gewährleisten, dass bei der zweizeitigen Bestrahlung der erfindungsgemäßen Komposite oder Kompositzusammensetzungen tatsächlich der erste Initiator nur durch Licht der ersten Wellenlänge aktiviert wird und der mindestens eine weitere Initiator nur durch Licht der zweiten Wellenlänge.

Es ist bekannt, dass Lichtquellen, die ein Maximum der Lichtintensität bei einer Wellenlänge von 465 nm aufweisen, immer auch einen kleinen Anteil von Licht mit kleinerer Wellenlänge als 430 nm abstrahlen. Dieser kurzwellige Lichtanteil ist ggf. ausreichend, den mindestens einen weiteren Initiator im erfindungsgemäßen Komposit oder in der erfindungsgemäßen Kompositzusammensetzung zu aktivieren, was sehr unerwünscht ist. Bei geeigneter Auswahl des optischen Elementes 24 wird jedoch Licht mit einer Wellenlänge von unter 435 nm aus dem Strahlengang entfernt und gelangt nicht zum Lichtleiter 25. Damit können diese unerwünschten Wellenlängen nicht über den Lichtleiter 25 zum Lichtaustrittsbereich 27 gelangen und auch nicht zu den erfindungsgemäßen Kompositen oder den erfindungsgemäßen Kompositzusammensetzungen gelangen und dort die unerwünschte Härtung initiieren.

Die erfindungsgemäße Polymerisationslampe 100 enthält zusätzlich eine Steuereinheit 29, welche automatisiert die mindestens zwei Lichtquellen 20, 21, 28 zum richtigen Zeitpunkt für die vorgesehenen Zeiten mit Strom versorgt. Der richtige Zeitpunkt ist abhängig von den Eigenschaften der verwendeten Initiatoren in den erfindungsgemäßen Kompositen oder Kompositzusammensetzungen.

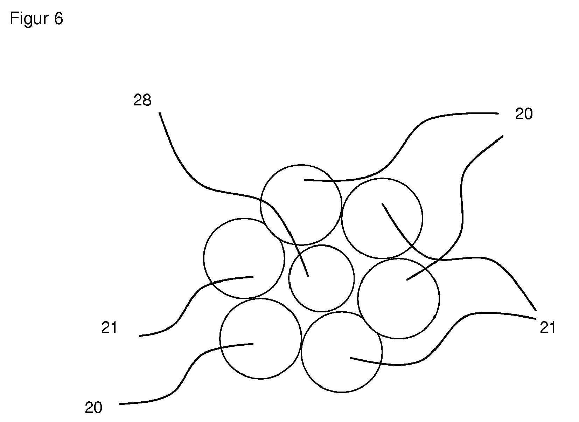

Wenn auch die Überlagerung des Lichtes verschiedener Wellenlängen mit einem dichroitischen Spiegel oder Strahlteilerprisma als optisches Element 24 eine identische Intensitätsverteilung ermöglicht, so kann man diesem Ziel auch dadurch nahe kommen, indem man die Lichtquellen 20, 21, 28 kreisförmig dicht beieinander anordnet, wobei benachbarte Lichtquellen immer die unterschiedlichen Wellenlängen abstrahlen. Es kann also bei der Polymerisationslampe 100 auf ein Strahlteilerprisma bzw. einen dichroidischen Spiegel als optisches Element 24 verzichtet werden, wenn die Lichtquellen 20, 21 und 28, die Licht mit verschiedenen Wellenlängen abstrahlen, kreisförmig angeordnet sind und benachbarte Lichtquellen immer Licht verschiedener Wellenlänge abstrahlen. Im Zentrum der kreisförmigen Anordnung der Lichtquellen 20 und 21 ist dabei eine weitere Lichtquelle 28 angebracht (s. Figur 6). Diese kreisförmige Anordnung mehrerer Lichtquellen 20, 21, 28 kann in einer Entfernung von wenigen mm von dem zu härtenden Komposit oder der zu härtenden Kompositzusammensetzung positioniert werden, oder vor dem Lichteintrittsbereich 26 eines Lichtleiters 25. Ein Beispiel für die Anordnung der Lichtquellen 20, 21, 28 ist in Figur 6 dargestellt. Im Zentrum des Kreises der Lichtquellen 20, 21 ist eine Lichtquelle 28 angeordnet, die Licht der Wellenlänge abstrahlt, von der die höhere Intensität benötigt wird. Bei der kreisförmigen Anordnung der Lichtquellen 20, 21, 28 gestaltet sich die Trennung der Lichtwellenlängen schwieriger, je nach verwendeten Lichtquellen und in Abhängigkeit von den Initiatoren in den Kompositen oder Kompositzusammensetzungen können passende Filter eingesetzt werden. Solche Filter können auch aus einem geeigneten Lack bestehen, der auf die Lichtquellen 20, 21, 28 aufgetragen wird.

Die erfindungsgemäßen Komposite oder Kompositzusammensetzungen umfassen polymerisierbare Harze, mineralische oder organische Füllstoffe und Farbpigmente. Ebenso umfassen sie geeignete Initiatoren (Aktivatoren für die Polymerisationsreaktion) wie beispielsweise Lucerin, Kampherchinon, (Di-) Benzoyl-Peroxid, Phenyl-Propandiol oder Acylgermanium-Verbindungen.

### Ausführungsbeispiele

### 1. Initiatoren:

Die erfindungsgemäßen Komposite oder Kompositzusammensetzungen sind dadurch gekennzeichnet, dass der erste Initiator Kampherchinon in einer Konzentration von 0,03 bis 0,1 % Gewichtsanteil bezogen auf den Harzanteil des Komposits oder der Kompositzusammensetzung ist. Der mindestens eine weitere Initiator ist Lucerin in einer Konzentration von 0,1 bis 2,0 % Gewichtsanteil bezogen auf den Harzanteil des Komposits oder der Kompositzusammensetzung. Beide Initiatoren sind in den erfindungsgemäßen Kompositen oder in den erfindungsgemäßen Kompositzusammensetzungen enthalten. Ein weiteres Ausführungsbeispiel für geeignete Initiatoren besteht darin, dass Kampherchinon in geringer Menge (beispielsweise 0,02% bezogen auf das Gewicht des Harzanteils des Komposits oder der Kompositzusammensetzung) dem Komposit oder der Kompositzusammensetzung beigegeben wird und Lucerin in einer Menge, die zur vollständigen Polymerisation ausreicht, beispielsweise 0,4% bezogen auf das Gewicht des Harzanteils des Komposits oder der Kompositzusammensetzung. Die Menge des Initiators Kampherchinon ist so bemessen, dass sie gerade ausreicht, die Anfangsphase der Polymerisation (Vorhärtung) einzuleiten. Das wird beispielsweise durch die Beleuchtung mit Licht mit der Wellenlänge von 460 nm für 15 Sekunden und mit einer Flächenenergiedichte von 800 mW/cm² erreicht. Dabei geliert das Komposit oder die Kompositzusammensetzung zu einer viskösen Masse, die jedoch nicht fest ist (Vorhärtung). Der zweite Initiator Lucerin spricht auf Licht der Wellenlänge von 460 nm nicht an, da die Quantenenergie zu gering ist. In diesem Zustand ist das Komposit oder die Kompositzusammensetzung schon zu einem großen Teil geschrumpft, kann aber mangels Festigkeit noch keine Schrumpfungsspannungen aufbauen, es ist also noch viskös. Das noch visköse Komposit oder die noch visköse Kompositzusammensetzung kann in die entstandenen Risse und Spalten nachfließen. Anschließend wird durch die Aktivierung des zweiten Initiators Lucerin das Komposit oder die Kompositzusammensetzung fertig ausgehärtet, beispielsweise durch die Beleuchtung mit Licht mit der Wellenlänge von 400 nm für 15 Sekunden und mit einer Flächenenergiedichte von 800 mW/cm² (Endhärtung). Da das gesamte Komposit oder die gesamte Kompositzusammensetzung durch die Aktivierung des ersten Initiators Kampherchinon bereits einen großen Teil der Polymerisationsschrumpfung durchgemacht hat, wird durch die Endhärtung durch die Aktivierung des zweiten Initiators Lucerin keine wesentliche Polymerisationsschrumpfung mehr verursacht. Ein solches zweizeitig polymerisierbares Komposit oder zweizeitig polymerisierbare Kompositzusammensetzung ist besonders für sog. Bulkfill-Komposite geeignet, die in Schichtstärken von bis zu 4 und mehr mm verarbeitet werden können.

In einem weiteren Ausführungsbeispiel sind die erfindungsgemäßen Komposite oder Kompositzusammensetzungen dadurch gekennzeichnet, dass der erste Initiator Lucerin in einer Konzentration von 0,03 bis 0,1 % Gewichtsanteil bezogen auf den Harzanteil des Komposits oder der Kompositzusammensetzung ist. Der mindestens eine weitere Initiator ist Kampherchinon in einer Konzentration von 0,1 bis 2,0 % Gewichtsanteil bezogen auf den Harzanteil des Komposits oder der Kompositzusammensetzung. Beide Initiatoren sind in den erfindungsgemäßen Kompositen oder Kompositzusammensetzungen vorhanden.

In allen Ausführungsbeispielen der erfindungsgemäßen Komposite oder Kompositzusammensetzungen ist der prozentuale Gewichtsanteil des mindestens einen weiteren Initiators mindestens doppelt so hoch ist wie der prozentuale Gewichtsanteil des ersten Initiators, unabhängig davon, welcher Initiator als erster Initiator verwendet wird und welcher Initiator als der mindesten einer weiterer Initiator.

Dem Fachmann ist ersichtlich, dass auch andere Kombinationen von Initiatoren verwendet werden können, ohne den Schutzumfang der Erfindung zu verlassen. Dem Fachmann ist ersichtlich, dass auch Kombinationen von drei oder mehr Initiatoren verwendet werden können, ohne den Schutzumfang der Erfindung zu verlassen.

### 2. Polymerisationslampe:

Eine Ausführungsform der erfindungsgemäße Polymerisationslampe wird in Figur 5 gezeigt. Es ist wesentlich, dass durch ein geeignetes optisches Element 24 die unerwünschten Wellenlängenanteile des Lichtes mit der ersten Wellenlänge daran gehindert werden, über den Lichteintrittsbereich 26, den Lichtleiter 25 und den Lichtaustrittsbereich 27 zum Komposit oder zur Kompositzusammensetzung zu gelangen. Das geeignete optische Element 24 in der Polymerisationslampe 100 kann ein frequenzselektives Strahlteilerprisma oder ein dichroitisches Strahlteilerprisma oder ein frequenzselektiven Spiegel oder ein dichroitischer Spiegel oder ein nichtfrequenzselektiver teildurchlässiger Spiegel sein.

Alternativ kann also der dichroitische Spiegel durch einen Spiegel ersetzt werden, der nur einen Teil des von den Lichtquellen 20, 21, 28 ausgesendeten Lichtes spiegelt und den anderen Teil des Lichtes durchlässt, und zwar unabhängig von der Wellenlänge des Lichtes. Dann erreicht nur ein entsprechend kleinerer Teil des von den Lichtquellen 20, 21, 28 ausgesendeten Lichtes den Lichteintrittsbereich 26 des Lichtleiters 25, entsprechend länger sind dann die Bestrahlungszeiten zur Erhärtung des Komposits oder der Kompositzusammensetzung. Außerdem muss gegebenenfalls durch zusätzliche Lichtfilter (nicht dargestellt) sichergestellt werden, dass nur Licht der gewünschten Wellenlängen den Lichtleiter 25 erreicht.

Die erfindungsgemäße Polymerisationslampe 100 ist dadurch gekennzeichnet, dass die Übergangswellenlänge des optischen Elements 24 zwischen 400 und 500 nm liegt, bevorzugt bei 435 +/- 10 nm.

Eine weitere Ausführungsform der erfindungsgemäßen Polymerisationslampe 100 weist eine spezielle geometrische Anordnung der Lichtquellen 20, 21, 28 auf, welche in Figur 6 gezeigt wird. Die Polymerisationslampe 100 weist eine Vielzahl von Lichtquellen 20, 21, 28 auf. Durch die geometrische Anordnung dieser Lichtquellen kann auf das dichroitische Strahlteilerprisma oder den dichroitischen Spiegel verzichtet werden. Die Lichtquellen 20 strahlen Licht mit einer ersten Wellenlänge aus, die Lichtquellen 21 strahlen Licht mit einer zweiten Wellenlänge aus. Die Lichtquelle 28 strahlt entweder Licht mit einer ersten Wellenlänge oder Licht mit einer zweiten Wellenlänge aus. Es ist wesentlich, dass zur Aktivierung des ersten Initiators in den erfindungsgemäßen Kompositen oder Kompositzusammensetzungen erst nur die Lichtquellen 20 Licht ausstrahlen, und anschließend erst zur Aktivierung des mindestens eines weiteren Initiators in den erfindungsgemäßen Kompositen oder Kompositzusammensetzungen die Lichtquellen 21 Licht ausstrahlen. Je nachdem, ob die Lichtquelle 28 Licht mit einer ersten Wellenlänge oder Licht mit einer zweiten Wellenlänge ausstrahlt, wird sie durch eine geeignete Steuereinheit 29 so gesteuert, dass sie entweder mit den Lichtquellen 20 gemeinsam Licht ausstrahlt oder mit den Lichtquellen 21.

Die erfindungsgemäße Polymerisationslampe 100 ist dadurch gekennzeichnet, dass die mindestens eine Lichtquelle 20, 21, 28 eine oder mehrere Leuchtdioden, Halogenlampen oder Laser sind.

Die erfindungsgemäße Polymerisationslampe 100 ist dadurch gekennzeichnet, dass die mindestens eine Lichtquelle 20, 21, 28 Licht im Wellenlängenbereich von 200 nm bis 800 nm ausstrahlt. Bevorzugt strahlen die Lichtquellen der erfindungsgemäßen Polymerisationslampe Licht im Wellenlängenbereich von 350 nm bis 600 nm aus. Besonders bevorzugt strahlen die Lichtquellen der erfindungsgemäßen Polymerisationslampe Licht im Wellenlängenbereich von 390 nm bis 470 nm aus.

Die erfindungsgemäße Polymerisationslampe 100 ist dadurch gekennzeichnet, dass die Polymerisationslampe mindestens eine Kondensor-Optik 23 umfasst, die zwischen der mindestens einen Lichtquelle 20, 21, 28 und dem optischen Element 24 angeordnet ist, um einen möglichst großen Teil des Lichtes von der mindestens einen Lichtquelle 20, 21, 28 in den Strahlengang einzubringen.

Dem Fachmann ist ersichtlich, dass auch andere Kombinationen von Bauteilen für eine Polymerisationslampe verwendet werden können, ohne den Schutzumfang der Erfindung zu verlassen.

### 3. Verfahren zur Härtung eines Komposits oder einer Kompositzusammensetzung:

Das erfindungsgemäße Verfahren zur Härtung eines erfindungsgemäßen Komposits oder einer erfindungsgemäßen Kompositzusammensetzung in einer Kavität mit einer erfindungsgemäßen Polymerisationslampe 100 ist durch folgende Schritte gekennzeichnet:
- Füllung einer Kavität mit einem erfindungsgemäßen Komposit oder einer erfindungsgemäßen Kompositzusammensetzung.
- Licht eines ersten Wellenlängenbereiches tritt am Lichtaustrittsbereich 27 des Lichtleiters 25 der Polymerisationslampe 100 aus, trifft auf das Komposit oder die Kompositzusammensetzung und aktiviert den ersten Initiator in dem Komposit oder in der Kompositzusammensetzung, so dass das Komposit oder die Kompositzusammensetzung in einen gelartigen Zustand übertritt und teilweise aushärtet (Vorhärtung). Während dieser Vorhärtung findet der größte Teil der Volumensschrumpfung des Komposits oder der Kompositzusammensetzung statt.
- Licht eines weiteren Wellenlängenbereiches tritt am Lichtaustrittsbereich 27 des Lichtleiters 25 der Polymerisationslampe 100 aus, trifft auf das Komposit oder die Kompositzusammensetzung und aktiviert den mindestens einen weiteren Initiator in dem Komposit oder in der Kompositzusammensetzung, so dass das Komposit oder die Kompositzusammensetzung vollständig aushärtet (Endhärtung). Während dieser Endhärtung findet nur noch ein sehr kleiner Teil der Volumensschrumpfung des Komposits oder der Kompositzusammensetzung statt.

Die beiden Schritte des erfindungsgemäßen Verfahrens können nacheinander, gleichzeitig oder überlappend ausgeführt werden.

Es ist wesentlich, dass die erfindungsgemäße Polymerisationslampe 100 zur Aktivierung des ersten Initiators in den erfindungsgemäßen Kompositen oder Kompositzusammensetzungen nur Licht mit einer ersten Wellenlänge ausstrahlt, damit nicht der mindestens eine weitere Initiator in den erfindungsgemäßen Kompositen oder Kompositzusammensetzungen bereits zu diesem Zeitpunkt aktiviert wird. Der mindestens eine weitere Initiator in den erfindungsgemäßen Kompositen oder Kompositzusammensetzungen darf erst durch das Licht mit einer zweiten Wellenlänge aktiviert werden, wenn die Aktivierung des ersten Initiators abgeschlossen ist. Damit erhält man ein zweizeitig polymerisierbares Komposit oder eine zweizeitig polymerisierbare Kompositzusammensetzung.

### Abbildungslegende

Figur 1 zeigt einen schematischen Längsschnitt durch einen Zahn mit einer Kavität 1, nachdem das kariöse Gewebe entfernt wurde. Es ist nur noch gesunder Zahnschmelz 2 und gesundes Dentin 3 vorhanden. Die Kavität 1 ist offen und noch ungefüllt. Die Zahnpulpa 4 ist nicht geschädigt und nicht eröffnet.
Figur 2 zeigt einen schematischen Längsschnitt durch einen Zahn mit einer Kavität, welche gemäß des Standes der Technik mit einem Komposit 10 aus dem Stand der Technik gefüllt wurde. Zwischen der Kavitätenoberfläche, gebildet durch den Zahnschmelz 2 und das Dentin 3, und dem Komposit 10 aus dem Stand der Technik befindet sich die Bondingschicht 5, welche aus dem gehärteten Haftvermittler besteht. Zur besseren Erkennbarkeit ist die Bondingschicht 5 wesentlich dicker dargestellt als sie in Wirklichkeit ist. Die Zahnpulpa 4 ist nicht geschädigt und nicht eröffnet.
Figur 3 zeigt einen schematischen Längsschnitt durch einen Zahn mit einer Kavität, welche mit einem erfindungsgemäßen Komposit oder einer erfindungsgemäßen Kompositzusammensetzung gefüllt wurde. Die Kompositzusammensetzung besteht in diesem Fall aus dem Komposit der Ausgleichsschicht 7 und dem Komposit der eigentlichen Füllung 6. Im Anschluss an die Kavitätenoberfläche befindet sich die Bondingschicht 5, welche aus dem gehärteten Haftvermittler besteht. Zur besseren Erkennbarkeit ist die Bondingschicht 5 wesentlich dicker dargestellt als sie in Wirklichkeit ist. Zwischen dem Komposit der eigentlichen Füllung 6 und der Bondingschicht 5 befindet sich das Komposit oder die Kompositzusammensetzung der Ausgleichsschicht 7, welches als Ausgleichsschicht für die Füllung der Risse und Spalten zwischen der Bondingschicht 5 und dem bereits gehärtetem Komposit der eigentlichen Füllung 6 fungiert. Die Härtung des Komposit der eigentlichen Füllung 6 stellt die Vorhärtung dar, während die Härtung des Komposit oder der Kompositzusammensetzung der Ausgleichsschicht 7 die Endhärtung darstellt.
Figur 4 zeigt einen schematischen Längsschnitt durch einen Zahn mit einer Kavität, welche mit einer erfindungsgemäßen Kompositzusammensetzung gefüllt wurde. Im Anschluss an die Kavitätenoberfläche befindet sich die Bondingschicht 5, welche aus dem gehärteten Haftvermittler besteht. Zur besseren Erkennbarkeit ist die Bondingschicht 5 wesentlich dicker dargestellt als sie in Wirklichkeit ist. Das Komposit oder die Kompositzusammensetzung der Ausgleichsschicht 7 bedeckt die Bondingschicht 5 wie auch in Figur 3 gezeigt. Das Komposit der eigentlichen Füllung ist eine Kompositzusammensetzung aus zwei verschiedenen Kompositen, welche übereinander geschichtet werden, nämlich einem selbstnivellierenden Komposit 8 und einem stopfbaren Komposit 9. Das stopfbare Komposit 9 ist besonders dafür geeignet, die Kaufläche zu rekonstruieren. Das selbstnivellierende Komposit 8 wird in die Kavität eingebracht und lichtgehärtet. Anschließend wird das stopfbare Komposit 9 in die Kavität eingebracht, dort modelliert, und somit die Kaufläche rekonstruiert. Es ist wesentlich, dass der Initiator im Komposit oder in der Kompositzusammensetzung der Ausgleichsschicht 7 ein anderer Initiator ist als die Initiatoren im selbstnivellierenden Komposit 8 und im stopfbaren Komposit 9. Bei den Initiatoren im selbstnivellierenden Komposit 8 und im stopfbaren Komposit 9 kann es sich um denselben Initiator handeln, oder um unterschiedliche Initiatoren.
Nach der Härtung des selbstnivellierenden Komposits 8 wird das stopfbare Komposit 9 gehärtet. Damit ist die Vorhärtung abgeschlossen. Das Komposit der Ausgleichsschicht 7 wird erst nach der Lichthärtung des stopfbaren Komposits 9 mit der dafür geeigneten Wellenlänge lichtgehärtet. Dabei handelt es sich um die Endhärtung.
Figur 5 zeigt eine Ausführungsform der erfindungsgemäßen Polymerisationslampe 100 mit Strahlzusammenführung. Die erste Lichtquelle 20 strahlt Licht einer ersten Wellenlänge ab, beispielsweise mit einem Intensitätsmaximum bei 400 nm. Die zweite Lichtquelle 21 strahlt Licht einer zweiten Wellenlänge ab, beispielsweise mit einem Intensitätsmaximum bei 460 nm. Das optische Element 24 spiegelt Licht mit einer Wellenlänge unter 435 nm und lässt Licht mit einer Wellenlänge über 435 nm durchtreten. Dadurch können die beiden Lichtquellen 20 und 21 störungsfrei überlagert werden und ihr Licht kann über einen Lichtleiter 25 geleitet werden. Es ist auch möglich, ein optisches Element 24 zu verwenden, das Licht mit einer Wellenlänge von mehr als 435 nm spiegelt und Licht mit einer Wellenlänge unter 435 nm durchtreten lässt. In diesem Fall müssen die Lichtquellen 20 und 21 in ihrer Position vertauscht werden, wodurch ebenfalls eine störungsfreie Überlagerung der beiden Lichtquellen 20 und 21 erreicht wird. Das optische Element 24 kann ein frequenzselektives Strahlteilerprisma oder ein dichroitisches Strahlteilerprisma oder ein frequenzselektiver Spiegel oder ein dichroitischer Spiegel oder ein nichtfrequenzselektiver teildurchlässiger Spiegel sein.

Für die Erfindung ist es unerheblich, ob ein dichroitischer Spiegel oder ein dichroitisches Strahlteilerprisma als optisches Element 24 verwendet wird. Wesentlich ist, dass der dichroitische Spiegel oder das dichroitische Strahlteilerprisma als optisches Element 24 eine Transmission der Wellenlängen von 444 bis 850 nm und eine Reflexion der Wellenlängen von 325 bis 425 nm gewährleistet.

Jeder Lichtquelle 20, 21 ist mindestens eine Kondensor-Optik 23 beigeordnet, welche dazu dient, einen möglichst großen Teil des Lichtes der Lichtquellen 20, 21 in den Strahlengang einzubringen. Die Kondensor-Optik 23 ist zwischen der jeweiligen Lichtquelle und dem optischen Element 24 bzw. dem Lichtleiter 25 angeordnet.

Über den Lichteintrittsbereich 26 und den Lichtleiter 25 wird das überlagerte Licht der Lichtquellen 20, 21, 28 zum Lichtaustrittsbereich 27 geleitet, von wo es auf die erfindungsgemäßen Komposite oder Kompositzusammensetzungen fällt und dort die mindestens zwei verschiedenen Initiatoren aktiviert. Durch die Aktivierung der Initiatoren wird die Polymerisation der erfindungsgemäßen Komposite oder Kompositzusammensetzungen ausgelöst und diese werden hart (Lichthärtung). Durch die Aktivierung des ersten Initiators wird die Vorhärtung ausgelöst, durch die Aktivierung des mindestens einen weiteren Initiators wird die Endhärtung ausgelöst.

Dem Fachmann ist bekannt, dass zu einer Polymerisationslampe weitere Bauteile dazugehören, wie beispielsweise elektrische Anschlussleitungen für die Lichtquellen, Kühlkörper, Steuerelektronik, Gehäuse und Chassisteile. Diese weiteren Bauteile sind nicht dargestellt.

Figur 6 zeigt eine bevorzugte geometrische Anordnung der Lichtquellen 20, 21, 28, wenn die erfindungsgemäße Polymerisationslampe 100 nicht mit einem optischen Element 24, wie beispielsweise einem Strahlteilerprisma oder dichroitischem Spiegel ausgestattet wird: Die Lichtquellen 20 und 21, vorzugsweise Leuchtdioden, sind im Kreis angeordnet, und zwar so, dass im Kreis benachbarte Lichtquellen immer Licht unterschiedlicher Wellenlänge abstrahlen. Im Zentrum befindet sich eine weitere Lichtquelle 28. Das Maximum des Wellenlängenbereiches des Lichtes der Lichtquelle 28 entspricht entweder dem Maximum des Wellenlängenbereiches des Lichtes der Lichtquelle 20, also beispielsweise 400 nm, oder es entspricht dem Maximum des Wellenlängenbereiches des Lichtes der Lichtquelle 21, also beispielsweise 460 nm. Über den Lichteintrittsbereich 26 und den Lichtleiter 25 wird das überlagerte Licht der Lichtquellen 20, 21, 28 zum Lichtaustrittsbereich 27 geleitet, von wo es auf die erfindungsgemäßen Komposite oder Kompositzusammensetzungen fällt und dort die mindestens zwei verschiedenen Initiatoren aktiviert. Durch die Aktivierung der Initiatoren wird die Polymerisation der erfindungsgemäßen Komposite oder Kompositzusammensetzungen ausgelöst und diese werden hart (Lichthärtung).

Alternativ kann auf einen Lichtleiter 25 mit einem Lichteintrittsbereich 26 und einem Lichtaustrittsbereich 27 verzichtet werden, womit das Licht der Lichtquellen 20, 21, 28 direkt auf die erfindungsgemäßen Komposite oder Kompositzusammensetzungen eingestrahlt wird.

### Bezugszeichenliste

- 1: Kavität (Zahnkavität)
- 2: Zahnschmelz
- 3: Dentin
- 4: Zahnpulpa
- 5: Bondingschicht
- 6: Komposit oder Kompositzusammensetzung der eigentlichen Füllung der Kavität, kann auch eine Kompositzusammensetzung sein, beispielsweise aus einem selbstnivellierenden Komposit und einem stopfbaren Komposit
- 7: Komposit oder Kompositzusammensetzung der Ausgleichsschicht
- 8: selbstnivellierendes Komposit oder Kompositzusammensetzung zur Füllung der Kavität
- 9: stopfbares Komposit oder Kompositzusammensetzung zur Füllung der Kavität
- 10: Komposit aus dem Stand der Technik zur Füllung der Kavität
- 20: erste Lichtquelle, Maximum der Lichtintensität bei 400 nm
- 21: zweite Lichtquelle, Maximum der Lichtintensität bei 460 nm
- 23: Kondensor-Optik
- 24: Optisches Element zur Filterung des Lichts der Lichtquellen 20, 21, 28, beispielsweise frequenzselektives Strahlteilerprisma oder dichroitisches Strahlteilerprisma oder frequenzselektiver Spiegel oder dichroitischer Spiegel oder ein nichtfrequenzselektiver teildurchlässiger Spiegel
- 25: Lichtleiter
- 26: Lichteintrittsbereich
- 27: Lichtaustrittsbereich
- 28: weitere Lichtquelle, Maximum der Lichtintensität bei 400 oder 460 nm
- 29: Steuereinheit
- 100: Polymerisationslampe

## Patentansprüche

1. Lichthärtbare Komposite oder Kompositzusammensetzungen geeignet zur Füllung einer Kavität, umfassend eine organische Kunststoffmatrix, anorganische und/oder organische Füllkörper, mindestens einen Koinitiator und einen ersten Initiator, der durch Licht eines ersten Wellenlängenbereiches aktivierbar ist, **dadurch gekennzeichnet, dass** die Komposite oder die Kompositzusammensetzungen mindestens einen weiteren Initiator umfassen, der durch Licht eines weiteren Wellenlängenbereiches aktivierbar ist, wobei der weitere Wellenlängenbereich unterschiedlich zum ersten Wellenlängenbereich ist.

2. Komposite oder Kompositzusammensetzungen nach Anspruch 1, **dadurch gekennzeichnet, dass** der erste und der mindestens eine weitere Initiator ausgewählt ist aus der Gruppe Kampherchinon, Lucerin, (Di-)Benzoyl-Peroxid, Phenyl-Propandiol oder Acylgermanium-Verbindungen.

3. Komposite oder Kompositzusammensetzungen nach Anspruch 2, **dadurch gekennzeichnet, dass** der erste Initiator Kampherchinon in einer Konzentration von 0,03 bis 0,1 % Gewichtsanteil bezogen auf den Anteil der organischen Kunststoffmatrix des Komposits oder der Kompositzusammensetzung und der zweite Initiator Lucerin in einer Konzentration von 0,1 bis 2,0 % Gewichtsanteil bezogen auf den Anteil der organischen Kunststoffmatrix des Komposits oder der Kompositzusammensetzung in dem Komposit oder in der Kompositzusammensetzung vorhanden ist.

4. Komposite oder Kompositzusammensetzungen nach Anspruch 2, **dadurch gekennzeichnet, dass** der erste Initiator Lucerin in einer Konzentration von 0,03 bis 0,1 % Gewichtsanteil bezogen auf den Anteil der organischen Kunststoffmatrix des Komposits oder der Kompositzusammensetzung und der zweite Initiator Kampherchinon in einer Konzentration von 0,1 bis 2,0 % Gewichtsanteil bezogen auf den Anteil der organischen Kunststoffmatrix des Komposits oder der Kompositzusammensetzung in dem Komposit oder der Kompositzusammensetzung vorhanden ist.

5. Komposite oder Kompositzusammensetzungen nach Anspruch 3 und 4, **dadurch gekennzeichnet, dass** der prozentuale Gewichtsanteil des ersten Initiators maximal halb so groß ist wie der prozentuale Gewichtsanteil des zweiten Initiators.

6. Komposite oder Kompositzusammensetzungen nach einem der Ansprüche 3 bis 5, **dadurch gekennzeichnet**, das der Initiator Lucerin ganz oder teilweise durch eine Acylgermanium-Verbindung ersetzt wird.

7. Komposite oder Kompositzusammensetzungen nach einem der Ansprüche 3 bis 5, **dadurch gekennzeichnet**, das der Initiator Lucerin ganz oder teilweise durch eine Propandiol-Verbindung ersetzt wird.

8. Polymerisationslampe (100) zur Aktivierung der Initiatoren in einem Komposit oder in einer Kompositzusammensetzung nach Anspruch 1, umfassend
- mindestens eine Lichtquelle (20, 21, 28),
- ein optisches Element (24), das zwischen der mindestens einen Lichtquelle (20, 21, 28) und dem Lichtleiter (25) angeordnet ist und das das Licht der mindestens einen Lichtquelle (20, 21, 28) so filtert, dass nur Anteile des Lichtes mit einer bestimmten Wellenlänge passieren können,
- eine Steuereinheit (29) zur zeitlichen Steuerung der mindestens einen Lichtquelle (20, 21, 28) und / oder des optischen Elementes (24) in Hinblick auf die abgestrahlte Lichtwellenlänge, den Zeitpunkt der Bestrahlung und die Dauer der Bestrahlung, so dass die Bestrahlung der Initiatoren in dem Komposit oder der Kompositzusammensetzung zeitlich gesteuert werden kann,
- einen Lichtleiter (25) mit einem Lichteintrittsbereich (26) und einem Lichtaustrittsbereich (27), der das Licht von der mindestens einen Lichtquelle (20, 21, 28) zum Komposit oder zu der Kompositzusammensetzung nach Anspruch 1 leitet,
**dadurch gekennzeichnet, dass** der erste und der mindestens eine weitere Initiator im Komposit oder in der Kompositzusammensetzung nach Anspruch 1 einzeln nacheinander oder gemeinsam aktivierbar sind.

9. Polymerisationslampe (100) nach Anspruch 8, **dadurch gekennzeichnet, dass** das optische Element (24) ein frequenzselektives Strahlteilerprisma oder ein dichroitisches Strahlteilerprisma oder ein frequenzselektiver Spiegel oder ein dichroitischer Spiegel oder ein nichtfrequenzselektiver teildurchlässiger Spiegel ist.

10. Polymerisationslampe (100) nach einem der Ansprüche 8 bis 9, **dadurch gekennzeichnet, dass** die mindestens eine Lichtquelle (20, 21, 28) Leuchtdioden, Halogenlampen oder Laser sind.

11. Polymerisationslampe (100) nach einem der Ansprüche 8 bis 10, **dadurch gekennzeichnet, dass** die mindestens eine Lichtquelle (20, 21, 28) Licht im Wellenlängenbereich von 200 nm bis 800 nm ausstrahlt.

12. Polymerisationslampe (100) nach Anspruch 8, **dadurch gekennzeichnet, dass** die Übergangswellenlänge des optischen Elements (24) zwischen 400 und 500 nm liegt, bevorzugt bei 435 +/- 10 nm.

13. Polymerisationslampe (100) nach einem der Ansprüche 8 bis 12, **dadurch gekennzeichnet, dass** die Polymerisationslampe mindestens eine Kondensor-Optik (23) umfasst, die zwischen der mindestens einen Lichtquelle (20, 21, 28) und dem optischen Element (24) angeordnet ist, um einen möglichst großen Teil des Lichtes von der mindestens einen Lichtquelle (20, 21, 28) in den Strahlengang einzubringen.

14. Verfahren zur Härtung eines Komposits oder einer Kompositzusammensetzung nach Anspruch 1 bis 7 mit einer Polymerisationslampe (100) nach Anspruch 8 bis 13 **gekennzeichnet durch** folgende Schritte:
- Licht eines ersten Wellenlängenbereiches tritt aus der Polymerisationslampe (100) aus, trifft auf das Komposit oder die Kompositzusammensetzung und aktiviert den ersten Initiator in dem Komposit oder in der Kompositzusammensetzung, so dass das Komposit oder die Kompositzusammensetzung teilweise aushärtet (Vorhärtung).
- Licht eines weiteren Wellenlängenbereiches tritt aus der Polymerisationslampe (100) aus, trifft auf das Komposit oder die Kompositzusammensetzung und aktiviert den mindestens einen weiteren Initiator in dem Komposit oder in der Kompositzusammensetzung, so dass das Komposit oder die Kompositzusammensetzung vollständig aushärtet (Endhärtung).

15. Verfahren nach Anspruch 14, **dadurch gekennzeichnet, dass** das Licht des weiteren Wellenlängenbereiches erst dann aus der Polymerisationslampe austritt, wenn das Licht des ersten Wellenlängenbereiches die Aktivierung des ersten Initiators vollständig abgeschlossen hat und die Vorhärtung abgeschlossen ist.
